(19) Europäisches Patentamt
European Patent Office
Office européen des brevets

(11) Veröffentlichungsnummer : **0 180 801**
**B1**

# (12) EUROPÄISCHE PATENTSCHRIFT

(45) Veröffentlichungstag der Patentschrift : **07.09.88**

(51) Int. Cl.⁴ : **C 07 C 51/56**, C 07 C 53/12, B 01 J 23/74

(21) Anmeldenummer : **85112823.1**

(22) Anmeldetag : **10.10.85**

## (54) Trägerkatalysator für die Herstellung von Monocarbonsäureanhydriden.

(30) Priorität : **07.11.84 DE 3440643**

(43) Veröffentlichungstag der Anmeldung : **14.05.86 Patentblatt 86/20**

(45) Bekanntmachung des Hinweises auf die Patenterteilung : **07.09.88 Patentblatt 88/36**

(84) Benannte Vertragsstaaten :
**AT BE CH DE FR GB IT LI NL**

(56) Entgegenhaltungen :
**EP-A- 0 102 561**
**FR-A- 2 347 097**
**US-A- 2 722 504**
**JOURNAL OF MOLECULAR CATALYSIS, Band 2, 1977, Seiten 223-226, Elsevier Sequoia S.A., Lausanne, CH; A.K. SMITH et al.: "The preparation and stability of nickel carbonyl complexes supported on a phosphinated silica"**

(73) Patentinhaber : **HOECHST AKTIENGESELLSCHAFT**
**Postfach 80 03 20**
**D-6230 Frankfurt am Main 80 (DE)**

(72) Erfinder : **Luft, Gerhard, Prof. Dr.**
**Ludwigstrasse 141a**
**D-6109 Mühltal (DE)**
Erfinder : **Ritter, Gebhard, Dr.**
**Johann-Peter-Hebelstrasse 11**
**D-7601 Schutterwald/Baden (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 180 801 B1**

## Beschreibung

Die Erfindung betrifft die Verwendung eines Trägerkatalysators, in dem eine Organosiliciumverbindung mit Alkoxy- oder Halogengruppen sowie mit Organostickstoff-, Organophosphor-, Organoarsen-, Organoschwefel-, Mercapto- oder Thioethergruppen als mehrfunktioneller Haftvermittler einerseits an das Trägermaterial und andererseits an eine Nickelverbindung gebunden ist, für die Herstellung von Monocarbonsäureanhydriden durch Carbonylierung der entsprechenden Ester oder Ether.

Die Herstellung derartiger Nickelverbindungen ist im Prinzip bekannt, vgl. A.K. Smith et al., J. Mol. Catalysis 2 (1977), S. 223-226.

Der erfindungsgemäß zu verwendende Trägerkatalysator ist insbesondere bestimmt für die Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor bei Temperaturen von 130 bis 400 °C und Drücken von 1-150 bar.

Ein derartiges, in der Gasphase mit einem Trägerkatalysator arbeitendes Verfahren ist bereits aus der DE-Offenlegungsschrift 24 50 965 und der JP-Offenlegungsschrift Nr. 47921/1975 bekannt, welche die bei den Flüssigphase-Verfahren auftretenden Nachteile, z. B. die schwierige Abtrennung und Rückführung von suspendiertem und teilweise gelöstem Katalysator und ggf. Promotor, vermeiden.

In beiden Schriften werden jedoch Gasphase-Verfahren nur mit solchen festen Trägerkatalysatoren beschrieben, welche durch Imprägnierung des Trägermaterials mit Edelmetallverbindungen enthaltenden Katalysatorlösungen hergestellt wurden. Es ist aber auf diese Weise nicht möglich, z. B. Organostickstoff- oder Organophosphorverbindungen mit dreibindigem Stickstoff bzw. Phosphor im Trägerkatalysator zu fixieren, was sich im allgemeinen auf die Katalysatoraktivität und die Selektivität der Reaktion ungünstig auswirkt.

Vorliegende Erfindung vermeidet auch diese Mängel durch Anwendung sog. mehrfunktioneller Haftvermittler (« Spacer »), in welche Promotoren der V. Hauptgruppe, z. B. Organylamine oder phosphine, bereits integriert sind. Mitt ihrer Hilfe ist es möglich, Nickelverbindungen fest an die Trägeroberfläche zu binden.

Die Carbonylierung von Alkoholen, Ethern und Estern mit einem Nickelverbindungen enthaltenden Katalysatorsystem wurde bereits in der US-A-2,729,651 beschrieben. Während dort überwiegend Nickel-Komplexe wie z. B. Triphenyl-ethyl-phosphoniumnickeltetrajodid eingesetzt werden, kann auch von z. B. Tetramethylammoniumjodid und Nickeljodid oder von Nickelpulver, Jod, Treithylamin und Ethyljodid ausgegangen werden. Obwohl Drücke bis zu 700 bar angewendet werden, sind die Raum-Zeit-Leistungen an Essigsäureanhydrid nur gering. Die Umsetzungszeiten liegen zwischen 5 und 26 Stunden. Sowohl der hohe Druck als auch die geringen Leistungen machen ein Arbeiten nach diesem Verfahren unwirtschaftlich.

Da das Reaktionsmedium korrosiv ist, so daß der Autoklav aus einer Legierung von Hastelloy B oder C oder aus Tantal hergestellt werden muß, bedürfen derartige Carbonylierungsverfahren eines sehr hohen Investitionsaufwands.

Das Verfahren der US-A-2,729,651 ist in der EP-A-102 561 als Stand der Technik abgehandelt. Rechnet man die dortigen Vergleichsbeispiele 1 und 2 auf Raum-Zeit-Ausbeuten um, so ergeben sich nur 22,3 g bzw. 30,1 g Acetanhydrid je g Nickel und Stunde. Eigentlicher Gegenstand der EP-A-102 561 ist jedoch die Verwendung eines Katalysatorsystems, das außer Nickelverbindungen noch Verbindungen des Vanadiums oder Niobs enthält, wodurch die Drucke auf etwa 100 bar gesenkt und die Leistungen gesteigert werden konnten.

Die erfindungsgemäße Verwendung des Trägerkatalysators kann weiterhin wahlweise und bevorzugt dadurch gekennzeichnet sein, daß

a) der Trägerkatalysator zusätzlich Metallverbindungen aus der 1. bis 3. Hauptgruppe oder der 4. bis 7. Nebengruppe des Periodensystems der Elemente als Promotoren enthält ;

b) im Trägerkatalysator die Organosiliciumverbindung als mehrfunktioneller Haftvermittler einerseits an das Trägermaterial und andererseits abwechselnd an die Nickelverbindung und an eine Metallverbindung aus der 5. bis 7. Nebengruppe des Periodensystems der Elemente gebunden ist ;

c) der mehrfunktionelle Haftvermittler eine Organosiliciumverbindung folgender allgemeiner Formeln ist :

$$R_n^1X_{3-n}Si{-}(CR_2^3)_m{-}Y \text{ oder} \qquad \text{I}$$

$$R_n^1X_{3-n}Si{-}(CR_2^3)_m{-}CHY_2 \text{ oder} \qquad \text{II}$$

$$[R_n^1X_{3-n}Si{-}(CR_2^3)_m]_2Z, \qquad \text{III}$$

wobei

$X = -Cl_4$, $-Br$ oder $-OR^2$;
$Y = -NR_2^4$, ein stickstoffhaltiger Arylrest, $-PR_2^4$, $-AsR_2^4$, $-SR^4$ oder $-SH$;
$Z = -NR^4-$, $-PR^4-$, $-AsR^4-$ oder $-S-$;
$R^1 = C_1$ bis $C_5$-Alkyl;
$R^2 = C_1$ bis $C_3$-Alkyl;
$R^3 = -H$, $C_1$ bis $C_5$-Alkyl oder $-C_6H_5$;
$R^4 = C_1$ bis $C_6$-Alkyl, $C_5$ bis $C_8$-Cycloalkyl oder $-C_6H_5$ oder $C_6H_5CH_2-$, die ggf. mit Halogen-, Methoxy-, Ethoxy- oder $C_1$ bis $C_3$-Alkyl substituiert sind;
n = 0 oder 1 oder 2;
m = 0 bis 8, vorzugsweise 1 bis 3.

d) der Trägerkatalysator ein anorganisches oxidisches Trägermaterial oder einen Aktivkohleträger enthält, deren restliche aktive Hydroxygruppen durch Veresterung oder Veretherung desaktiviert wurden;

e) der Trägerkatalysator zusammen 0,01 bis 50 Gew%, vorzugsweise 0,1 bis 20 Gew%, Nickelverbindung, Haftvermittler und ggf. andere Nichtedelmetallverbindungen enthält.

Als Katalysatorträger kommen bevorzugt anorganische Oxide wie z. B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle infrage, welche BET-Oberflächen von 1-1 000 $m^2/g$, vorzugsweise 30-400 $m^2/g$, haben und stets noch OH-Gruppen aufweisen. Diese OH-Gruppen reagieren mit der oder den funktionellen Gruppen X des Haftvermittlers unter Bildung von Sauerstoffbrücken zwischen Träger und Haftvermittler. Die Promotoren der 5. oder 6. Hauptgruppe sind im Haftvermittler chemisch gebunden und bilden selbst eine seiner funktionellen Gruppen, an welche die Nickelverbindungen, ggf. abwechselnd mit Metallverbindungen aus der 5. bis 7. Nebengruppe, insbesondere von Vanadium, Chrom, oder Rhenium angelagert werden. Hierbei können diese Nickel- und ggf. anderen Nichtedelmetallverbindungen Brücken zwischen den einzelnen, am Träger fixierten Haftvermittler-Molekülen bilden.

Es ist ein Vorteil der Erfindung, daß die zur Steigerung der Katalysatoraktivität und der Selektivität notwendigen Promotoren aus der Hauptgruppe V oder VI des Periodensystems der Elemente eine funktionelle Gruppe Y oder Z in mehrfunktionellen Haftvermittlern bilden und somit bis zur maximalen Konzentration, die durch die Anzahl der OH-Gruppen auf der Trägeroberfläche bestimmt ist, fixiert werden können. Deshalb entfällt eine Abtrennung und Rückführung dieser z. B. Organostickstoff- oder Organophosphorpromotoren. Das Verfahren zur Herstellung von Monocarbonsäureanhydriden, katalysiert mit dem erfindungsgemäß zu verwendenden Trägerkatalysator, weist gegenüber den eingangs beschriebenen bekannten Verfahren, welche bereits in der Gasphase mit einem Trägerkatalysator arbeiten, höhere Katalysatoraktivitäten und Selektivitäten auf. Außerdem enthalten die erfindungsgemäß zu verwendenden Trägerkatalysatoren keine teuren Edelmetalle aus der Gruppe VIII des Periodensystems der Elemente.

Der erfindungsgemäß zu verwendende Trägerkatalysator dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1-4 C-Atomen bedeutet.

In den allgemeinen Formeln für die Organosiliciumverbindungen, welche als Haftvermittler (Spacer) infrage kommen, bedeutet X vorzugsweise $-OR^2$ und insbesondere Methoxy und Ethoxy. Sofern n nicht ohnehin null ist, bedeutet $R^1$ bevorzugt einen unverzweigten Alkylrest, insbesondere Methyl, Ethyl oder Propyl.

Die Trägermaterialen wurden bereits genannt; als Mischoxide kommen z. B. $Cr_2O_3-Al_2O_3$, $WO_3-Al_2O_3$, $MgO-Al_2O_3$, $SiO_2-Al_2O_3$ oder $ZrO_2-Al_2O_3$ infrage. Der Trägerkatalysator enthält bevorzugt 0,01 bis 5 Gew% Nickel und wird in einer Korngröße von 1 bis 20 mm eingesetzt.

Als Nickelverbindungen kommen bei der Herstellung des Trägerkatalysators z. B. folgende Verbindungen infrage:

$$Ni(CO)_4,\ [P(C_6H_5)_3]_2Ni(CO)_2,\ NiCl_2,\ Ni(C_8H_{12})_2$$

Als Metallverbindungen aus der 1. bis 3. Hauptgruppe oder der 4. bis 7. Nebengruppe des Periodensystems, vorzugsweise des Li, Na, Mg, Ca, Al, Ti, Zr, V, Cr, W, Re, können z. B. Hydroxide, Carbonate, Carbonyle, Hydride, Halogenide und andere Salze eingesetzt werden. Diese Metallverbindungen können z. B. als Lösung durch Imprägnierung auf den Katalysatorträger aufgebracht werden.

Zur Herstellung des erfindungsgemäß eingesetzten Trägerkatalysators muß zuerst der mehrfunktionelle Haftvermittler (Organosiliciumverbindung) bereitgestellt werden. Dieser ist entweder im Handel erhältlich oder kann nach oder in Analogie zu Literaturangaben dargestellt werden. Im allgemeinen wird jetzt an diese Haftvermittler, und zwar an die Promotorgruppe Y oder Z, welche ein Element aus der 5. oder 6. Hauptgruppe enthält, in an sich bekannter Weise eine der genannten Nickelverbindungen und ggf. eine der genannten Metallverbindungen aus der 5. bis 7. Nebengruppe angelagert. Anschließend erfolgt die an sich bekannte reaktive Anlagerung des nickelhaltigen Zwischenprodukts an die Hydroxygruppen des Trägermaterials unter Austritt einer Gruppe X als Verbindung XH (z. B. HCl, HBr oder $R^2OH$).

Dies wird durch 24- bis 100-stündiges Erhitzen am Rückfluß der in einem unpolaren Lösemittel (z. B. Benzol, Toluol, Xylol) suspendierten Komponenten bis zur Entfärbung erreicht.

Andererseits kann man auch zuerst den mehrfunktionellen Haftvermittler (Organosiliciumverbindung) in an sich bekannter Weise reaktiv unter Austritt einer Gruppe X als Verbindung XH an die Hydroxygruppen des Trägermaterials und erst anschließend in ebenfalls an sich bekannter Weise die Nickelverbindung und ggf. eine der genannten Metallverbindungen aus der 5. bis 7. Nebengruppe an die Promotorgruppe Y oder Z des Zwischenprodukts anlagern. Alle Einzelheiten ergeben sich aus der Katalysatorbeschreibung.

Besonders bei diskontinuierlichem und in der Anfahrphase bei kontinuierlichem Betrieb ist es zwecks Erhöhung der Selektivität und Unterdrückung von Nebenreaktionen sinnvoll, die restlichen OH-Gruppen auf der Oberfläche des Katalysatorträgers, welche nicht mit den funktionellen Gruppen X des Haftvermittlers reagiert haben, zu desaktivieren. Dies kann z. B. durch Silylierung mit Trimethylchlorsilan, Methylierung mit Methyljodid oder Acetylierung mit Essisgsäureanhydrid geschehen.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 Mol, vorzugsweise 1 bis 100 Mol, je 1 Mol Jod(verbindung) oder Brom(verbindung). Die Temperatur der Reaktionszone wird so gewählt, daß das Reaktionsgemisch bei jedem beliebigem Umsatz gasförmig vorliegt. Bevorzugt wird die Temperatur zwischen 170 und 250 °C gewählt. Der bevorzugte Druck liegt zwischen 10 und 40 bar.

Die Verweilzeit des Reaktionsgemisches am festen Trägerkatalysator der Erfindung beträgt 1 bis 1 000 s, bevorzugt 1 bis 180 s. Die Umsetzung kann in einem vorzugsweise senkrecht angeordneten und mit Trägerkatalysator gefüllten Strömungsrohr oder auch in einem Rühr- oder Schüttelautoklaven erfolgen, der den Trägerkatalysator enthält.

Das aus der Carbonylierungszone abströmende Reaktionsgemisch ist gasförmig und enthält Kohlenmonoxid, Methyljodid, Essigsäureanhydrid, nicht-umgesetztes Methylacetat oder Dimethylether und ggf. geringe Mengen Essigsäure. Das gasförmige Reaktionsgemisch wird abgekühlt, wobei Essigsäureanhydrid und ggf. Essigsäure auskondensieren. Die nicht kondensierten Gase wie CO, $CH_3I$, Methylacetat oder Dimethylether werden in die Reaktionszone zurückgeführt, wobei die umgesetzten Anteile von Ester oder Ether sowie CO fortlaufend ersetzt werden. Die einfache Abtrennung der Anhydride durch Kühlung des abströmenden Reaktionsgemisches und Rückführung der nicht kondensierbaren Gase stellt einen wesentlichen Vorteil des mit dem erfindungsgemäß zu verwendenden Trägerkatalysator durchgeführten Verfahrens dar, da dies ohne komplizierte Trennoperationen erfolgen kann. Der erfindungsgemäß zu verwendende Trägerkatalysator wird nicht verunreinigt und verbleibt in der Reaktionszone, was den gesamten Verfahrensablauf bedeutend vereinfacht.

## Beispiel

Herstellung des Katalysators

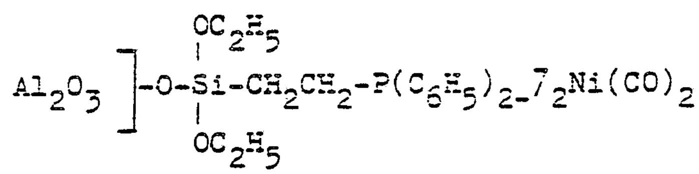

Aluminiumoxid wurde zwecks Aktivierung bei 200 °C und 0,1 bis 0,2 mbar 10 h getrocknet. Die Herstellung des Katalysators wurde in Gegenwart von Stickstoff unter Ausschluß von Sauerstoff und Wasser durchgeführt, wobei sämtliche Reagenzien zuvor über Molekularsieb 4 A getrocknet worden waren.

Zu 3,2 g aktiviertem Aluminiumoxid (99 % $Al_2O_3$) mit einem Korndurchmesser von 3 mm, einer inneren Oberfläche nach BET von 125 $m^2/g$ und einem Porenvolumen von 0,9 ml/g gab man 65 mg (4,4 mg Ni) der Verbindung $[(C_2H_5O)_3Si—CH_2CH_2P(C_6H_5)_2]_2Ni(CO)_2$, gelöst in 40 ml Xylol, unter Rühren hinzu und brachte die Mischung zum Sieden. Nach 72 h Rückfluß war die gelbliche Lösung vollständig entfärbt. Nach Abtrennung des Lösemittels wurde der Katalysator in die Soxhlet-Apparatur überführt. Nach 12 h Soxhlet-Extraktion mit Benzol wurde der Katalysator bei 1,33 mbar und 85 °C acht Stunden getrocknet.

Zur Unterdrückung von Nebenreaktionen und Verbesserung der Selektivität wurde der Katalysator abschließend mit Trimethylchlorsilan behandelt.

$$Al_2O_3]\text{-}OH + Cl\text{-}Si(CH_3)_3 \xrightarrow[-HCl]{58^\circ C} Al_2O_3]\text{-}O\text{-}Si(CH_3)_3$$

Zu diesem Zweck wurde er bei Raumtemperatur mit Trimethylchlorsilan vollständig bedeckt. Diese

Suspension wurde zum Sieden erhitzt und so lange unter Rückfluß gekocht bis keine Gasentwicklung mehr auftrat. Anschließend ließ man die Suspension abkühlen, trennte den Katalysator von der Flüssigkeit ab und trocknete ihn bei 85 °C und 1,33 mbar 12 Stunden lang.

Die eingeengten Lösemittel waren frei von Nickel. Der Nickelgehalt des so hergestellten Katalysators beträgt 0,13 Gew%.

Zur Herstellung der Zwischenverbindung $[(C_2H_5O)_3Si—CH_2CH_2P(C_6H_5)_2]_2Ni(CO)_2$ aus $(C_2H_5O)_3SiCH_2CH_2P(C_6H_5)_2$ und $Ni(CO)_4$ unter Abspaltung von CO-Gas siehe A.K. Smith et al., J. mol. Catal. 2 (1977)S. 223-226. Zur Herstellung von $(C_2H_5O)_3$ $SiCH_2CH_2P(C_6H_5)_2$ aus Triethoxyvinylsilan und Diphenylphosphin unter UV-Belichtung siehe H. Niebergall, Makromol. Chem. 52 (1962), S. 218-227.

Verwendung des Katalysators

a) 2 ml (1,86 g) Essigsäuremethylester, 0,5 ml (1,14 g) Methyljodid und 3,15 g des Katalysators wurden in einem 0,25 Liter fassenden Autoklaven aus korrosionsfreiem Edelstahl (Hastelloy C) mit Kohlenmonoxid bei 20 bar CO-Druck und 200 °C umgesetzt. Nach 1 h Reaktionsdauer ergab sich eine Raum-Zeit-Ausbeute von 140 g $Ac_2O/g_{Ni} \cdot h$.

Die Ausbeute von $Ac_2O$, bezogen auf den eingesetzten Ester, betrug 22 % bei einer Selektivität von 92 %.

b) 1,86 g Dimethylether 0,5 ml (1,14 g) Methyljodid und 3,15 g des Katalysators wurden in dem Autoklaven mit Kohlenmonoxid bei 20 bar CO-Druck und 200 °C umgesetzt. Die Raum-Zeit-Ausbeute bezüglich Essigsäureanhydrid betrug 30 g $Ac_2O/g_{Ni} \cdot h$. Der Versuch wurde über 5 h durchgeführt.

Die Ausbeute von $Ac_2O$, bezogen auf den eingesetzten Ether, betrug 6 % bei einer Selektivität von 19 %.

**Patentansprüche**

1. Verwendung eines Trägerkatalysators, in dem eine Organosiliciumverbindung mit Alkoxy- oder Halogengruppen sowie mit Organostickstoff-, Organophosphor-, Organoarsen-, Organoschwefel-, Mercapto- oder Thioethergruppen als mehrfunktioneller Haftvermittler einerseits an das Trägermaterial und andererseits an eine Nickelverbindung gebunden ist, für die Herstellung von Monocarbonsäureanhydriden durch Carbonylierung der entsprechenden Ester oder Ether.

2. Verwendung nach Anspruch 1, dadurch gekennzeichnet, daß der Trägerkatalysator zusätzlich Metallverbindungen aus der 1. bis 3. Hauptgruppe oder der 4. bis 7. Nebengruppe des Periodensystems der Elemente als Promotoren enthält.

3. Verwendung nach Anspruch 1 oder 2, dadurch gekennzeichnet, daß im Trägerkatalysator die Organosiliciumverbindung als mehrfunktioneller Haftvermittler einerseits an das Trägermaterial und andererseits abwechselnd an die Nickelverbindung und an eine Metallverbindung aus der 5. bis 7. Nebengruppe des Periodensystems der Elemente gebunden ist.

4. Verwendung nach einem der Ansprüche 1-3, dadurch gekennzeichnet, daß der mehrfunktionelle Haftvermittler eine Organosiliciumverbindung folgender allgemeiner Formeln ist :

$$R_n^1X_{3-n}Si—(CR_2^3)_m—Y \text{ oder} \qquad \text{I}$$

$$R_n^1X_{3-n}Si—(CR_2^3)_m—CHY_2 \text{ oder} \qquad \text{II}$$

$$[R_n^1X_{3-n}Si—(CR_2^3)_m]_2Z, \qquad \text{III}$$

wobei

$X = —Cl$, $—Br$ oder $—OR^2$ ;

$Y = —NR_2^4$, ein stickstoffhaltiger Arylrest, $—PR_2^4$, $—AsR_2^4$, $—SR^4$ oder $—SH$ ;

$Z = —NR^4—$, $—PR^4—$, $—AsR^4—$ oder $—S—$ ;

$R^1 = C_1$ bis $C_5$-Alkyl ;

$R^2 = C_1$ bis $C_3$-Alkyl ;

$R^3 = —H$, $C_1$ bis $C_5$-Alkyl oder $—C_6H_5$ ;

$R^4 = C_1$ bis $C_6$-Alkyl, $C_5$ bis $C_8$-Cycloalkyl oder $—C_6H_5$ oder $C_6H_5CH_2—$, die ggf. mit Halogen-, Methoxy-, Ethoxy- oder $C_1$ bis $C_3$-Alkyl substituiert sind ;

$n = 0$ oder 1 oder 2 ;

$m = 0$ bis 8, vorzugsweise 1 bis 3.

5. Verwendung nach einem der Ansprüche 1-4, dadurch gekennzeichnet, daß der Trägerkatalysator ein anorganisches oxidisches Trägermaterial oder einen Aktivkohleträger enthält, deren restliche aktive Hydroxygruppen durch Veresterung oder Veretherung desaktiviert wurden.

6. Verwendung nach einem der Ansprüche 1-5, dadurch gekennzeichnet, daß der Trägerkatalysator zusammen 0,01 bis 50 Gew%, vorzugsweise 0,1 bis 20 Gew%, Nickelverbindung, Haftvermittler und ggf. andere Nichtedelmetallverbindungen enthält.

7. Verwendung nach einem der vorhergehenden Ansprüche, dadurch gekennzeichnet, daß der Trägerkatalysator die Formel

Träger 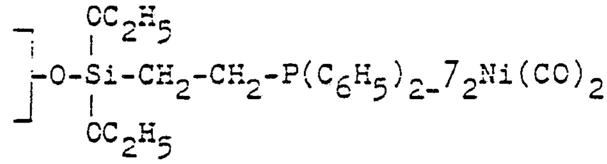

aufweist.

## Claims

1. The use of a supported catalyst in which an organosilicon compound containing alkoxy or halogen groups and containing organonitrogen, organophosphorus, organoarsenic, organosulfur, mercapto or thioether groups is bonded, as a polyfunctional adhesion promoter, on the one hand to the support material and on the other hand to a nickel compound, for the preparation of a monocarboxylic anhydride by carbonylation of the appropriate ester or ether.

2. The use as claimed in claim 1, wherein the supported catalyst additionnally contains, as promoters, metal compounds from main groups 1 to 3 or sub-groups 4 to 7 of the Periodic Table of the Elements.

3. The use as claimed in claim 1 or 2, wherein, in the supported catalyst, the organosilicon compound, as a polyfunctional adhesion promoter, is bonded on the one hand to the support material and on the other hand alternately to the nickel compound and to a metal compound from the sub-groups 5 to 7 of the Periodic Table of the Elements.

4. The use as claimed in one of claims 1-3, wherein the polyfunctional adhesion promoter is an organosilicon compound of the following formula :

$$R_n^1X_{3-n}Si{-}(CR_2^3)_m{-}Y \text{ or} \qquad \text{I}$$

$$R_n^1X_{3-n}Si{-}(CR_2^3)_m{-}CHY_2 \text{ or} \qquad \text{II}$$

$$[R_n^1X_{3-n}Si{-}(CR_2^3)_m]_2Z, \qquad \text{III}$$

where

$X = -Cl$, $-Br$ or $-OR^2$ ;

$Y = -NR_2^4$, a nitrogen-containing aryl radical, $-PR_2^4$, $-AsR_2^4$, $-SR^4$ or $-SH$ ;

$Z = -NR^4-$, $-PR^4-$, $-AsR^4-$ or $-S-$ ;

$R^1 = C_1$ to $C_5$-alkyl ;

$R^2 = C_1$ to $C_3$-alkyl ;

$R^3 = -H$, $C_1$ to $C_5$-alkyl or $-C_6H_5$ ;

$R^4 = C_1$ to $C_6$-alkyl, $C_5$ to $C_8$-cycloalkyl or $C_6H_5$ or $C_6H_5CH_2$-, which are optionally substituted by halogen, methoxy, ethoxy or $C_1$ to $C_3$-alkyl ;

$n = 0$ or 1 or 2 ; and

$m = 0$ to 8, preferably 1 to 3.

5. The use as claimed in one of claims 1-4, wherein the supported catalyst contains an inorganic oxidic support material or an activated charcoal support whose residual active hydroxyl groups have been deactivated by esterification or etherification.

6. The use as claimed in one of claims 1-5, wherein the supported catalyst contains in total 0.01 to 50 % by weight, preferably 0.1 to 20 % by weight, of nickel compound, adhesion promoter and, where appropriate, other non-noble metal compounds.

7. The use as claimed in one of the previous claims, wherein the supported catalyst has the formula

Träger 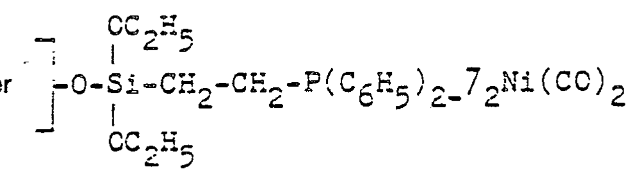

## Revendications

1. Utilisation d'un catalyseur sur support dans lequel un composé d'organosilicium contenant des

groupes alcoxy ou halogéno ainsi que des groupes organo-azotés, organo-phosphorés, organo-arséniés, organo-soufrés, mercapto ou thioéthers, en tant qu'agent d'adhérence polyfonctionnel, est fixé d'une part à la matière de support et d'autre part à un composé de nickel, pour la préparation d'anhydrides d'acides monocarboxyliques par carbonylation des esters ou éthers correspondants.

2. Utilisation selon la revendication 1, caractérisé en ce que le catalyseur sur support contient en outre des composés métalliques du 1$^{er}$ au 3$^{e}$ groupe principal ou du 4$^{e}$ au 7$^{e}$ sous-groupe de la classification périodique des éléments en tant qu'activateurs.

3. Utilisation selon la revendication 1 ou 2, caractérisée en ce que, dans le catalyseur sur support, le composé d'organosilicium, en tant qu'agent d'adhérence polyfonctionnel, est fixé d'une part à la matière de support et d'autre part, selon le cas, au composé du nickel ou à un composé métallique du 5$^{e}$ au 7$^{e}$ sous-groupe de la classification périodique des éléments.

4. Utilisation selon l'une des revendications 1 à 3, caractérisée en ce que l'agent d'adhérence polyfonctionnel est un composé d'organosilicium répondant aux formules générales suivantes :

$$R_n^1X_{3-n}Si-(CR_2^3)_m-Y \text{ ou} \qquad \text{I}$$

$$R_n^1X_{3-n}Si-(CR_2^3)_m-CHY_2 \text{ ou} \qquad \text{II}$$

$$[R_n^1X_{3-n}Si-(CR_2^3)_m]_2Z, \qquad \text{III}$$

dans lesquelles

$X = -Cl$, $-Br$ ou $-OR^2$ ;

$Y = -NR_2^4$, un groupe aryle contenant de l'azote, $PR_2^4$, $-AsR_2^4$, $-SR^4$ ou $-SH$ ;

$Z = -NR^4-$, $-PR^4-$, $-AsR^4-$ ou $-S-$ ;

$R^1$ = alkyle en $C_1$-$C_5$ ;

$R^2$ = alkyle en $C_1$-$C_3$ ;

$R^3$ = $-H$, alkyle en $C_1$-$C_5$ ou $C_6H_5$ ;

$R^4$ = alkyle en $C_1$-$C_6$, cycloalkyle en $C_5$-$C_8$ ou $C_6H_5$ ou $C_6H_5CH_2$-, éventuellement substitués par des halogènes, des groupes méthoxy, éthoxy ou alkyle en $C_1$-$C_3$ ;

n = 0 ou 1 ou 2 ;

m = 0 à 8, de préférence 1 à 3.

5. Utilisation selon l'une des revendications 1 à 4, caractérisée en ce que le catalyseur sur support contient une matière de support du type oxyde minéral ou du type charbon actif dont les groupes hydroxy actifs résiduels ont été désactivés par estérification ou éthérification.

6. Utilisation selon l'une des revendications 1 à 5, caractérisée en ce que le catalyseur sur support contient au total de 0,01 à 50 % en poids, de préférence de 0,1 à 20 % en poids, de composé du nickel, d'agent d'adhérence et le cas échéant d'autres composés de métaux non nobles.

7. Utilisation selon l'une des revendications qui précèdent, caractérisée en ce que le catalyseur sur support répond à la formule :